(19)

Europäisches
Patentamt
European
Patent Office
Office européen
des brevets

(11) **EP 4 407 045 A1**

(12) **EUROPEAN PATENT APPLICATION**

(43) Date of publication:
**31.07.2024  Bulletin 2024/31**

(21) Application number: **23382070.3**

(22) Date of filing: **27.01.2023**

(51) International Patent Classification (IPC):
**C12Q 1/6883** (2018.01)    **G01N 33/53** (2006.01)

(52) Cooperative Patent Classification (CPC):
**C12Q 1/6883;** C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Universidad del Pais Vasco - Euskal Herriko Unibertsitatea (UPV/EHU)**
  **20018 Donostia-San Sebastián (ES)**
- **Servicio de Salud de Castilla-la Mancha (SESCAM)**
  **45007 Toledo (ES)**
- **Administración General Comunidad Autónoma de Euskadi**
  **01010 Gasteiz (ES)**
- **Consorcio Centro de Investigación Biomédica en Red**
  **28029 Madrid (ES)**

(72) Inventors:
- **Bujanda  Fernández de Pierola, Luis**
  **Donostia - San Sebastián (ES)**
- **García Etxebarria, Koldo**
  **Donostia - San Sebastián (ES)**
- **Lucendo Villarin, Alfredo**
  **Tomelloso (ES)**
- **Castellanos Rubio, Ainara**
  **Donostia - San Sebastián (ES)**
- **Bilbao Catala, Jose Ramón**
  **Donostia - San Sebastián (ES)**
- **Sebastián De la Cruz, Maialen**
  **Donostia - San Sebastián (ES)**

(74) Representative: **Pons**
**Glorieta Rubén Darío 4**
**28010 Madrid (ES)**

Remarks:
The complete document including Reference Table(s) and the Sequence Listing(s) can be downloaded from the EPO website

(54) **NON-INVASIVE METHOD FOR THE DIAGNOSIS OF EOSINOPHILIC ESOPHAGITIS**

(57)    The present invention discloses a non-invasive method for the diagnosis of eosinophilic esophagitis (EoE), wherein said method is based on the quantification of expression levels of biomarkers in saliva samples isolated from a subject. The biomarkers used in the present method are CDH26, KCNJ2 and PLD1. These biomarkers showed differential levels of expression in saliva samples of subjects suffering from EoE when compared to control subjects who do not suffer from said disease. Moreover, data of the expression levels of CDH26, KCNJ2 and PLD1 can be combined with the clinical data of the subject leading to an increase in the specificity and sensibility of the method. Thus, the present invention discloses a non-invasive, accurate and reliable method for the diagnosis of EoE.

A

**Logistic regression**
(*CDH26 + KCNJ2 + PLD1*)

B

**ROC of *CDH26 + KCNJ2 + PLD1***

p=0.0002
AUC=0.7764

**FIG. 4**

EP 4 407 045 A1

## Description

The present invention relates to the diagnosis of eosinophilic esophagitis (EoE) by means of the detection and quantification of the expression levels of EoE biomarker genes present in the saliva of subjects. Thus, the invention is within the biotechnology and biomedicine sectors.

## BACKGROUND ART

Eosinophilic esophagitis (EoE) is an inflammatory disease characterized by a high number of eosinophils in the esophagus. It causes symptoms such as difficulty swallowing, esophageal food impaction, vomiting, heartburn, or regurgitation. In addition, in children, EoE causes food refusal, chest or abdominal pain, irritability and low weight gain. Such variable symptoms could be confused with other digestive disorders, such as gastroesophageal reflux disease. Although the exact cause of the disease is unknown, EoE is considered a particular form of food allergy, with a multifactorial origin to which genetic and environmental factors contributes.

EoE is increasing in incidence and prevalence at a high rate, which may be due to increased recognition and awareness of symptoms, but also to environmental and etiological changes (Dellon, E. S. and Hirano, I, Gastroenterology 2018; 25 154(2): 319-332). Due to this increase, EoE has become a significant cause of upper gastrointestinal morbidity in children and adults.

One of the problems related to EoE is the delay in diagnosis and consequent delay in treatment. Late diagnosis of the disease and persistence of a chronic inflammatory response may cause fibrous remodeling of the esophagus, with collagen deposition in the wall of the esophagus and formation of stenosis, the frequency of which is directly related to the time of evolution of symptoms and delay in diagnosis. Thus, an early diagnosis of the disease can positively affect its clinical course. A better understanding of the progressive nature of the disease, its burden of symptoms over time, and the impact of current therapies on symptom resolution are crucial to direct current clinical practice.

Current diagnostic criteria for EoE require endoscopy in addition to an esophageal biopsy to confirm the presence of infiltration of eosinophils in the esophageal mucosa in patients with compatible symptoms. Obtaining at least 6 biopsies generally from at least two esophageal locations is recommended, focusing on areas with endoscopic mucosal abnormalities. The cut-off of 15 eosinophils/per high power field (HPF) is considered as the diagnostic threshold in a patient with symptoms of esophageal dysfunction. Despite being the most reliable method for EoE diagnosis, endoscopic biopsies are an invasive procedure that also causes the diagnostic process extends over time (Dellon et al., Gastroenterology. 2018; 155(4): 1022-1033).

Several works have been carried out to find biomarkers of EoE and methods of diagnosis and/or prognosis of EoE that are less invasive and simpler and faster to perform. Document US2017006711 discloses several possible biomarkers for EoE, as well as a diagnostic method where the concentration of eotaxin-3 in the blood of a patient, where an elevated level of eotaxin-3 compared with reference values is indicative of EoE. Document US20160304960 discloses a method to differentiate between EoE and chronic esophagitis through the analysis of the gene expression levels in a patient's esophageal biopsy. The document WO2015142739 discloses a diagnostic and/or prognostic method consisting of determine the patient's genotype for genetic variants that are associated with EoE development.

However, the current methods for the diagnosis in the prior art are based on invasive strategies, they need highly specialized equipment and personnel, and/or the analysis of complicated and cumbersome data. Thus, according to the above, there is a need in the state to the art to develop an alternative diagnostic method that allows the detection of EoE quickly, simply, and non-invasively.

## DESCRIPTION OF THE INVENTION

The inventors have detected several EoE biomarkers whose expression levels change significantly in subjects suffering from EoE, being said change detectable, quickly and efficiently, in a saliva sample isolated from the subject.

After assessing different biomarkers, individual evaluation of the diagnostic performance of the three significant genes using ROC analysis yielded an area under the curve (AUC) value of 0.72 for *CDH26* (p=0.0022), 0.69 for *KCNJ2* (p=0.0059), and 0.64 for *PLD1* (p=0.036). Based on these findings, the inventors set a biomarker signature including the three biomarkers *CDH26, KCNJ2* and *PLD1.* The combination of *CDH26, KCNJ2* and *PLD1* yielded a ROC-plot AUC of 0.78 (p=0.0002) and the predictive model generated achieved 80.5% sensitivity and 66.7% specificity, leading to an improvement in the diagnosis when combining the three biomarkers with respect to the use of any of them alone. Therefore, the inventors found that the combination *CDH26, KCNJ2* and *PLD1* biomarkers surprisingly showed a significant differential expression between saliva samples of controls and active EoE patients, being useful in the diagnostic of EoE.

In addition, the inventors also realized that if these three biomarkers are combined with clinical data from the subject, such as age, sex and the presence of related atopies, the diagnosis of EoE significantly improved. Therefore, the prediction model comprising biomarker signature expression levels (*CDH26, KCNJ2* and *PLD1)* and the clinical data yielded a ROC-plot AUC of 0.95 (p<0.0001) with 90.2% sensitivity and 91.7% specificity.

Therefore, the inventors have developed an accurate and reliable non-invasive diagnostic method for EoE based on the expression levels of CDH26, KCNJ2 and PLD1 genes alone or in combination with the clinical data from the subject.

Thus, one aspect of the present invention relates to the use of the expression levels of CDH26, KCNJ2 and PLD1 genes as biomarkers for the *in vitro* diagnosis of EoE in a saliva sample isolated from a subject, hereinafter "diagnostic use of the invention".

In the present invention the term "eosinophilic esophagitis" or "EoE" refers to an inflammatory disease characterized by an elevated number of eosinophils in the esophagus. It causes varied symptoms, such as difficulty swallowing (dysphagia), food impactions, nausea, vomiting, heartburn, or a burning sensation that spreads from the epigastrium towards the chest and cervical region, reduced intake, chest or abdominal pain, regurgitation, or weight loss. Without clinical intervention, chronic inflammation that characterizes EoE may develop progressive fibrostenosis and may include esophageal lumen narrowing. At the tissue level, EoE is characterized by dense infiltration of white blood cells of the eosinophilic type in the epithelial lining of the esophagus. EoE is usually determined by an allergic reaction to food ingested. Eosinophils are inflammatory cells that release a variety of chemical signals that inflame the surrounding esophageal tissue.

The term "CDH26 gene" or "CDH26 biomarker" refers to the human gene (Ensembl accession number: ENST00000348616.9) that codes for the cadherin like-26 protein (protein CDH26). The cadherin like-26 protein is a member of the cadherin protein family. Cadherins are a family of calcium-dependent adhesion molecules that mediate cell-cell adhesion in all solid tissues and modulate a wide variety of processes, including cell polarization, migration and differentiation. In a particular embodiment, the CDH26 biomarker comprises, or consist of, a nucleotide sequence with a sequence identity of at least 70%, 75%, 80%, 85%, 86%, 87%, 88%, 89%, 90%, 91%, 92%, 93%, 94%, 95%, 96%, 97%, 98% or 99% with the nucleotide sequence SEQ ID NO: 1. In a particular embodiment, the CDH26 biomarker comprises, or consist of, a nucleotide sequence with a sequence identity of 100% with SEQ ID NO:1.

The term "KCNJ2 gene" or "KCNJ2 biomarker" refers to refers to the human gene (Ensembl accession number: ENST00000243457.4) that codes for the Potassium Inwardly Rectifying Channel Subfamily J Member 2 protein. The Potassium Inwardly Rectifying Channel Subfamily J Member 2 is a protein channel that has a greater tendency to allow potassium to flow into a cell rather than out of a cell and probably participates in establishing action potential wave form and excitability of neuronal and muscle tissues. In a particular embodiment, the KCNJ2 biomarker comprises, or consist of, a nucleotide sequence with a sequence identity of at least 70%, 75%, 80%, 85%, 86%, 87%, 88%, 89%, 90%, 91%, 92%, 93%, 94%, 95%, 96%, 97%, 98% or 99% with the nucleotide sequence SEQ ID NO: 2. In a particular embodiment, the KCNJ2 biomarker comprises, or consist of, a nucleotide sequence with a sequence identity of 100% with SEQ ID NO: 2.

The term "PLD1 gene" or "PLD1 biomarker" refers to the human gene (Ensmbl accession number: ENST00000351298.9) that encodes for the protein PLD1. The protein PLD1 is a phosphatidylcholine-specific phospholipase which catalyses the hydrolysis of phosphatidylcholine in order to yield phosphatidic acid and choline. In a particular embodiment of the use of the invention, the PLD1 biomarker comprises, or consist of, a nucleotide sequence with at least 85% identity with the nucleotide sequence SEQ ID NO: 3, preferably 70%, 75%, 80%, 86%, 87%, 88%, 89%, 90%, 91%, 92%, 93%, 94%, 95%, 96%, 97%, 98% or 99%. In a more particular embodiment, the PLD1 biomarker comprises, or consist of, a nucleotide sequence with a 100% identity with SEQ ID NO: 3.

In the context of the present invention, the term "sequence identity" or "identity" is understood to mean the degree of similarity between two nucleotides or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programs, for example, BLASTN, BLASTX, and BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions for the functionality of the protein are evolutionarily neutral mutations which do not affect its global structure or its functionality. Thus, amino acids or nucleotides sequences with identities of at least 70% can be considered to retain the same properties as the sequence to which they refer, i.e., they are significantly overexpressed in saliva samples of subjects with active EoE, being functionally equivalent variants of SEQ ID NO: 1, or SEQ ID NO: 2, or SEQ ID NO: 3. Any of these functionally equivalent variants can be used to put into practice the present invention.

Hereinafter, "CDH26 biomarker", "KCNJ2 biomarker" and "PLD1 biomarker" are referred as "biomarkers of the invention".

The term "biomarker", as used herein, refers to a gene whose transcription and/or translation produces a substance (a RNA, a cDNA, or a protein) whose presence can be objectively determined and/or quantified in a saliva sample and used as an indicator of the presence/absence of the disease. In the present invention, the disease is EoE.

Thus, the set of biomarkers of the invention taken together conform a biomarker signature useful for the diagnosis of EoE in a saliva sample of a subject. Thus, the biomarker signature of the invention comprises the CDH26, KCNJ2 and PLD1 biomarkers.

The expression "expression levels of biomarker(s)" or "expression levels of a set of biomarkers" in the present invention refers to the process by which information encoded by nucleic acids in a gene or genes is transcribed and/or translated into functional products. Such transcription and/or translation can originate ribonucleotide acids (RNA) functional as messenger RNA (mRNA), transfer RNA (tRNA), small nuclear RNA or complementary DNA (cDNA), or proteins. Thus, "expression levels" refers to the amount of said proteins, RNAs, cDNA or fragments of proteins, RNAs and cDNAs, that are present in a sample or obtained from a sample and that can be quantified by units of mass per volume (i.e., milligrams/millilitre) or molar units by volume (i.e., millimoles/millilitre). The expression "expression levels of a biomarker signature" as used in the present invention, refers to the expression profile of the set of biomarkers taken together that conform a biomarker signature.

Therefore, in another particular embodiment of the diagnostic use of the invention, the expression levels of CDH26, KCNJ2 and PLD1 biomarkers are quantified by the expression levels of the messenger RNA (mRNA) of said biomarkers, or a fragment of said mRNA, of the complementary DNA (cDNA) of said biomarkers, or a fragment of said cDNA, or of the protein encoded by said biomarkers, or a fragment of said protein.

In the present invention, the terms "mRNA fragment" or "cDNA fragment" refers to the nucleotide sequence of the genes associated with a biomarker that comprises one or more nucleotides absent from the 3' and/or 5' ends with respect to the complete nucleotide sequence of the gene associated with the biomarker.

Similarly, in the present invention the expression "fragment of the protein encoded by the biomarker" refers to the amino acid sequence of the protein encoded by the biomarkers of the invention comprising one or more amino acids absent from its amino terminal end and/or carboxyl terminal end with respect to the complete amino acid sequence of the proteins encoded by the biomarkers of the invention.

As explained in the beginning of the present description, the biomarkers of the invention are useful for the diagnosis of EoE. In the present invention, "diagnosis" is understood as the procedure that identifies a particular disease, nosological entity, syndrome or any health disturbance or disease condition, through the analysis of a series of clinical parameters or symptoms characteristic of said disease, and which distinguishes it from other diseases with similar clinical pictures. In the present invention, the disease to identify is EoE, and the parameter used for that purpose is the level of expression of the biomarkers of the invention.

Moreover, the inventors also found that if the expression levels of the biomarkers of the invention in saliva are combined with the clinical data from the subject, the diagnosis of EoE is unexpectedly improved.

Therefore, in a preferred embodiment of the diagnostic use of the invention, the expression levels of CDH26, KCNJ2 and PLD1 biomarkers are combined with clinical data from the subject for the diagnosis of EoE.

In the present invention, the term "clinical data" refers to data related to the physical and health state of a subject including, but not limited to, age, sex, personal history of diseases such as reflux disease, obesity, admission to the emergency room, surgical injuries, taking drugs, allergies, use of toxins (alcohol, tobacco, and others). In a preferred embodiment of the use of the invention, the clinical data correspond to the age of the subject, sex of the subject and the presence or absence of an atopy or atopies.

As the skilled person knows, atopy refers to a genetic tendency to develop allergen-specific IgE on exposure to environmental allergens, developing allergic diseases or conditions such as allergic rhinitis, asthma and atopic dermatitis, food allergy and non-food allergy. Atopy is typically associated with heightened immune responses to common allergens, especially inhaled allergens and food allergens (European Academy of Allergy and Clinical Immunology. Position paper: a revised nomenclature for allergy. EAACI position statement. Allergy. 2001 ;56:813-24). Therefore, the term "atopy", as used in the present invention, refers to an immunological condition in which the immune system of a subject is more likely to trigger an immune response in the presence of a substance that is recognized as a threat, wherein said substance is considered as an allergen. Conditions related to atopy are preferably asthma, rhinitis, dermatitis, food allergy and non-food allergy.

The term "asthma", as used in the present invention, refers to a respiratory condition marked by attacks of spasm in the bronchi of the lungs, causing difficulty in breathing, triggered by an allergen that contacts with the airways.

The term "rhinitis", as used in the present invention, refers to a reaction occurs that causes nasal congestion, runny nose, sneezing, and itching. Most types of rhinitis are caused by an inflammation and are associated with symptoms in the eyes, ears, or throat.

The term "dermatitis", as used in the present invention, refers in general terms to the common skin irritation.

The term "food allergy", as used in the present invention, refers to the situation when the body's immune system reacts unusually to specific foods. Symptoms of a food allergy can affect different areas of the body at the same time, being the most common symptoms: an itchy sensation inside the mouth, throat or ears; a raised itchy red rash (urticaria, or "hives"); swelling of the face, around the eyes, lips, tongue and roof of the mouth (angioedema); or even vomiting.

The term "non-food allergy", as used in the present invention, refers to the situation when body's immune system reacts unusually to substances like chemicals, dust mites, grasses, weeds or trees, insect bites, latex, medicines and pets. Symptoms of mild or moderate non-food allergic reactions include rash, swelling, tingling, stomach pain and hay fever.

The term "subject", as used in the present invention, refers to any animal, preferably a mammal and includes, but is not limited to, domestic animals and farm animals, primates, and humans. In a preferred embodiment, the subject is a mammal, preferably a human of any sex, age or race. Preferably, the subject is suspected of having EoE, when the subject shows compatible symptoms with EoE.

In the present invention, "sample" is understood as a part or small amount of a thing that is considered representative of the whole and that is taken or separated from it to submit it to study, analysis or experimentation. In the present invention, said study, analysis or experimentation refers to the determination of the expression levels of the biomarkers of the invention in a sample of saliva.

In a further aspect, the present invention refers to an *in vitro* method for the diagnosis of EoE in a subject, hereinafter "the method of the invention", wherein said method comprises the following steps:

> a) quantifying the expression levels of CDH26, KCNJ2 and PLD1 biomarkers in a saliva sample isolated from the subject; and
> b) comparing the expression levels of each biomarker of step a) with reference values for said biomarkers;

wherein an increase in the expression levels of CDH26, KCNJ2 and PLD1 biomarkers on the sample from the subject compared to the reference values is indicative that the subject is suffering from, or is predisposed to suffering from, EoE.

The term "quantifying" in the method of the invention refers to the determination of quantity, number, or concentration in unit volume, of the expression levels of the biomarkers of the invention.

Thus, as used in the method of the invention, the expression "quantifying the expression levels" of step a) refers to the method of reporting and/or determining the amount of the expression levels of the biomarkers of the invention by detecting and/or determining the amount of the mRNA, or a fragment of the mRNA, cDNA, or a fragment of the cDNA, or of the protein or a fragment of the protein, encoded by the biomarkers of the invention. Thus, in a particular embodiment of method of the invention, step (a) is carried out by quantifying the expression levels of CDH26, KCNJ2 and PLD1 biomarkers by quantification of the expression levels of the messenger RNA (mRNA) of said biomarkers, or a fragment of said mRNA, the complementary DNA (cDNA) of said biomarkers, or a fragment of said cDNA, or the protein encoded by said biomarkers, or a fragment of said protein. The terms "CDH26 biomarker", "KCNJ2 biomarker" and "PLD1 biomarker" (biomarkers of the invention) have been defined for the diagnostic use of the invention and said definitions, together with the preferred embodiments thereof, applicable to the present invention.

Likewise, the terms "mRNA fragment", "cDNA fragment" and "protein fragment" have been defined above in relation to the diagnostic use of the invention and said definitions are valid for the present aspect.

If the quantification of the expression levels of the biomarkers of the invention is going to be carried out from the cDNA or mRNA, it is first necessary to extract the nucleic acid from the saliva sample isolated from the subject. For this purpose, the saliva sample can be treated to physically or mechanically disrupt the structure of the tissue or cell, releasing the intracellular components in an aqueous or organic solution to isolate and prepare the nucleic acids. Nucleic acids are extracted by procedures known to those skilled in the art and commercially available.

Once the nucleic acids are extracted, the quantification of the expression levels of biomarkers is carried out. Virtually any conventional method can be used within the framework of the present invention to detect and quantify the levels of mRNA of the biomarkers. As an illustration, not limiting, the mRNA levels of these biomarkers can be quantified by employing conventional methods, for example, methods that comprise the amplification of mRNA and the quantification of the product of the amplification of said mRNA, such as electrophoresis and staining, or alternatively, by Southern blot and use of appropriate probes, northern blot and use of specific probes of the mRNA of the biomarker of interest or its cDNA corresponding, mapping with S1 nuclease, RT-PCR, hybridization, microarrays, etc.

Similarly, the levels of the cDNA corresponding to the mRNA of the biomarkers of the invention can also be quantified by employing conventional techniques; in this case, the method of the invention includes a synthesis step of cDNA by corresponding reverse transcription (RT) of mRNA followed by amplification and quantification of the corresponding product of the amplification of said cDNA.

In a preferred embodiment of the diagnostic method of the invention, alone or in combination with all or each one of the previous preferred embodiments, the quantification of mRNA or cDNA levels is carried out by polymerase chain reaction. In a preferred embodiment, the quantification of expression levels is performed by a quantitative polymerase chain reaction (PCR), a DNA or RNA array, o RNA-Seq or Massive Sequencing applied to the study of RNA. In a more preferred embodiment of the diagnostic method of the invention the quantification of the levels of messenger RNA is carried out by reverse transcription polymerase chain reaction (RT-PCR).

If the quantification of the expression levels of the biomarkers of the invention is going to be carried out from the protein encoded by the gene associated with each biomarker, then the saliva sample isolated from the subject has to be treated to extract the proteins. Methods for extracting or isolating proteins are known to those skilled in the art. matter and are commercially available.

Protein levels can be quantified by any method which allows detecting and quantifying said protein in a sample of a subject. By way of illustration, not limitation, the levels of said protein can be quantified, for example, by using tests with the ability to bind to the target protein and the subsequent quantification of the complexes formed.

The term "antibody" is understood as a glycoprotein of the gamma globulin type that forms part of the humoral immune system that specifically binds to an antigen. The key terms, as used here, include monoclonal tests, polyclonal studies, recombinant fragment studies, combibodies, Fab and scFv fragments of experiments, as well as the ligand binding domains.

The antibodies used in these assays may or may not be labeled. The terms "label" or "labeled" refer to a composition capable of producing a detectable signal indicative of the presence of the label molecule. Illustrative examples of suitable labels include, without limitation, radioisotopes, chromophores of nucleotides, enzymes, substrates, fluorescent molecules, chemiluminescent fractions, magnetic particles, bioluminescent residues and the like. As such, a label is any composition detectable by spectroscopy, photochemistry, biochemical, immunochemical, electrical, optical or chemical. There is a wide variety of known assays that can be used in the present invention, which use unlabeled antibodies (primary antibody) and labeled antibodies (secondary antibody); these techniques include Western blot or Western blot, ELISA (enzyme-linked immunosorbent assay RIA (radioimmunoassay), competitive EIA (competitive enzyme-linked immunosorbent assay), DAS-ELISA (sandwich ELISA with double antibody), immunocytochemical and immunohistochemical techniques, techniques based on the use of biochips or protein microarrays that include specific antibodies or assays based on colloidal precipitation in formats such as test strips. Other ways to detect and quantify proteins, including chromatography techniques affinity, ligand binding assays, mass spectrometry, etc. When using an immunological method, any antibody or reagent known to bind to the target protein with high affinity to detect the amount of it. Examples of antibodies or reagents capable of binding said target protein include, but not limited to, polyclonal serum, hybridoma supernatants, or antibodies monoclonal, antibody fragments, Fv, Fab, Fab' and F(ab')2, scFv, diabodies, triabodies, tetrabodies and humanized antibodies. In a preferred embodiment, the quantification of the protein levels is carried out by Western blotting or enzyme-linked immunosorbent assay

Once the levels of the biomarkers of the invention have been quantified, the method comprises a step b) comprising comparing the expression levels of each biomarker with reference values for said biomarkers.

The term "reference values" in step b) of the diagnostic method of the invention refers to the values of the expression levels of the biomarkers of the invention, CDH26, KCNJ2 and PLD1 in subjects without EoE. These values can be obtained from the quantification of the expression levels of the biomarkers of the invention in saliva samples isolated from subjects not suffering from EoE.

The expression "comparing the expression levels of each biomarker of step a) with reference values for said biomarkers" refers to the process or procedure of examining, relating and verifying the values obtained for the expression levels of the biomarkers of the invention, and comparing them to the values that are obtained from the subjects who do not suffer from the disease.

After comparing the expression levels of each biomarker of the invention with reference values as explained above, can be concluded that an increase in the expression levels of CDH26, KCNJ2 and PLD1 biomarkers on the sample from the subject compared to reference values is indicative that the subject is suffering from, or is predisposed to suffering from, EoE.

The term "increase" refers to an increase of the expression levels values obtained for a subject suffering from or being susceptible to suffer from EoE when these values are compared to the reference values, that is, with values of a subject who does not suffer from EoE. In particular, the "increase" of the expression levels of each of the biomarkers of the invention are at least 0.1 times, 0.5 times, 1 times, 5 times, 10 times, 20 times, 30 times, 40 times, 50 times, 60 times, 70 times, 80 times, 90 times, 100 times or even more with regarding the expression levels of the biomarkers of the invention in a subject who does not suffer from EoE.

Thus, the expression "an increase in the expression levels of CDH26, KCNJ2 and PLD1 biomarkers on the sample from the subject compared to the reference values is indicative that the subject suffers from, or is predisposed to, suffering from EoE" implies that the subject is classified into a group of subjects that suffer from, or are predisposed to suffering from, EoE.

The present invention may be put into practise by means of a computer. Thus, in a further aspect, the invention refers to a computer implemented method (CIM) for the diagnosis of EoE, hereinafter referred as "the computer implemented method of the invention", the computer performing the steps comprising:

a) receiving inputted subject data comprising values for the expression levels of CDH26, KCNJ2 and PLD1 biomarkers in a saliva sample isolated from the subject; and
b) calculating an EoE score, wherein EoE score is a result of applying a probability function to a linear combination of the expression levels;

c) determining that the subject suffers from, or is predisposed to suffering from, EoE, if the EoE score is greater than or equal to a reference score.

The computer implemented method of the invention comprises a first step a) comprising receiving inputted subject data comprising values for the expression levels of CDH26, KCNJ2 and PLD1 biomarkers in a saliva sample isolated from the subject.

The terms "subject", "expression levels", "CDH26, KCNJ2 and PLD1 biomarkers" and "sample", as well as how to quantify the expression level of the biomarkers, have been defined above for previous inventive aspect and they are applicable to CIM of the invention. The inputted subject data, i.e, the expression levels of CDH26, KCNJ2 and PLD1 biomarkers, may be received from a device that carries out the quantification of said biomarkers as previously defined in the method of the invention.

Next, in a step b), the computer implemented method comprises calculating an EoE score, wherein EoE score is a result of applying a probability function to a linear combination of the expression levels of CDH26, KCNJ2 and PLD1 biomarkers in a saliva sample isolated from the subject.

The term "linear combination" refers to an expression constructed from a set of variables by multiplying each variable by a constant. In a preferred embodiment the linear combination, referred as to "z", is represented by the formula (1):

$$(1) \quad z = \alpha 1 * P1 + \alpha 2 * P2 + \alpha 3 * P3 \dots \alpha n * Pn$$

In the present invention, the terms *"P1, P2 P3,..., Pn"* of the formula (1) represent the variables that correspond to the expression levels CDH26, KCNJ2 and PLD1 biomarkers, wherein each "P" corresponds to the expression levels of a determined biomarker, e.g. P1 corresponds to the expression level of CDH26. The values *"$\alpha 1$, $\alpha 2$, $\alpha 3$, $\alpha n$"* correspond to each of constants that multiply each of the aforementioned variables. The values of the constants can be obtained empirically, or by means of machine learning or artificial intelligence allowing the comparison of data obtained from subjects suffering from EoE and comparing it to data obtained from healthy subjects.

The term "probability function" refers to a nonnegative function that gives the probability that the outcome associated with a variable or set of variables will occur, e.g the outcome is the presence or absence of a disease associated with the expression levels of a marker or set of markers.

Therefore, as the skilled person understands, the computer implemented method of the invention estimates the probability that the subject suffers from, or is predisposed to suffering from, EoE. More specifically, the computer implemented method makes it possible to determine that the subject has a higher probability of suffering from, or being predisposed to being suffering from, EoE, if the EoE score is greater than or equal to a reference score.

In a preferred embodiment of the computer implemented method of the invention, the probability function of step b) is represented by the formula (2):

$$(2) \quad EoE\ score = \ 1/(1 + e^{\wedge}(-z))$$

wherein z is the linear combination of the expression levels of CDH26, KCNJ2 and PLD1 biomarkers. The EoE score ranges from 0 to 1.

Finally, in step c), the computer implemented method comprises determining that the subject suffers from, or is predisposed to suffering from EoE, if the EoE score is greater than or equal to a reference score.

In the present invention, the terms "reference score" or "EoE reference core", used interchangeably, refer to a predetermined score or value that is statistically predictive of the presence or diagnosis of EoE. The reference score can be also understood as a cut-off value or threshold. The reference score is previously determined empirically, or by means of machine learning or artificial intelligence allowing the comparison of data obtained from subjects suffering from EoE and comparing it to data obtained from healthy subjects

In addition, the inventors of the present invention have found out that taking into account clinical data from the subject, the sensibility and specificity of the EoE diagnosis is improved. The material and methods for collecting the clinical data of subjects are common general knowledge and once these data are collected, they may be introduced by the technician into the computer (i.e. in the inputted clinical data from the subject) that carries out the computer implemented method of the invention.

Thus, in a preferred embodiment of the computer implemented method of the invention, step a) further comprises receiving inputted clinical data from the subject. Example of clinical data include, without being limited to, age, sex, personal history of diseases such as reflux disease, obesity, admission to the emergency room, surgical injuries, taking drugs, allergies, use of toxins (alcohol, tobacco, and others). Nevertheless, in a more preferred embodiment of the computer implemented method of the invention, said clinical data comprise at least the age, sex and/or the presence or absence of atopies, being the atopies selected from the list consisting of asthma, rhinitis, dermatitis, food allergy and non-food allergy. If the mentioned clinical data are used, the EoE score, that is calculated in step b) of the computer implemented method of the invention, is then the result of applying a probability function to a linear combination of the expression levels of CDH26, KCNJ2 and PLD1 biomarkers together with the inputted clinical data from the subject using the formula (2).

Therefore, in a preferred embodiment, the terms *"P1, P2 P3,..., Pn"* of the linear combination according to the formula (1), represent the variables that correspond to the expression levels CDH26, KCNJ2 and PLD1 biomarkers and the clinical data of the subject, wherein each "P" corresponds to the expression levels of a determined biomarker or each of the clinical data of the subject. The values *"α1, α2, α3, αn"* correspond to each of constants that multiply each of the aforementioned variables.

In a more preferred embodiment of the computer implemented method of the invention, the "z" value of the formula (2) for the calculation of EoE score in step b), is calculated according to the formula (3):

$$(3) \qquad Z = -2,2303 + (2,0286 * \text{CDH26}) + (5,809 * \text{KCNJ2}) + (3,210 * \text{PLD1})$$

$$- (0,049 * \text{Age}) + (2,1235 * \text{Sex}) + (2,7705 * \text{Presence of atopies})$$

In a preferred embodiment, the value of the reference score of the step c) of the computer implemented method is 0.5898, leading to a %91.67 of specificity and 90.24% of sensibility; or 0.815, leading to a 100% of specificity and 80.49% of sensibility.

For the lineal combination, numeric values are allocated to clinical data:

- the age values are considered the exact age when the saliva sample is collected;
- sex numeric values are divided in 1 for male and 0 for female; and
- a value of 1 is given when the subject presents any of the atopies selected from the list consisting of asthma, rhinitis, dermatitis, food allergy and non-food allergy.

On the other hand, the computer that carries the computer implemented method of the invention, in addition to the above-mentioned steps a) to c), may also perform a step of outputting a report related to the diagnosis of EoE. Thus, in a preferred embodiment of the computer implemented method of the invention, the computer further performs a step of outputting a report that comprises a suggested diagnosis of EoE. The report may be presented on an output unit such as a display or a printer suggesting a positive or negative diagnosis of EoE.

Additionally, the report may further comprise a treatment recommendation. The term "treatment", as used in the present invention, refers to combat the effects caused as a result of a disease or a pathological condition in a subject (preferably mammal, and more preferably a human) comprising:

i) Inhibiting the disease or pathological condition i.e. stopping its development;
ii) Alleviating the disease or disease state, i.e. causing regression of the disease or the pathological state or its symptomatology;
iii) Stabilizing the disease or pathological state.

The computer comprises a storage component (i.e., memory) for storing data, wherein the storage component has instructions for determining the diagnosis of the subject stored therein; a computer processor for processing data, wherein the computer processor is coupled to the storage component and configured to execute the instructions stored in the storage component; and output unit or display component for displaying information regarding the diagnosis of the subject.

The storage component may be of any type capable of storing information accessible by the processor, such as a hard-drive, memory card, ROM, RAM, DVD, CD-ROM, USB Flash drive, write-capable, and read-only memories. The processor may be any well-known processor, such as processors from Intel Corporation. Alternatively, the processor may be a dedicated controller such as an ASIC.

The instructions may be any set of instructions to be executed directly (such as machine code) or indirectly (such as scripts) by the processor. In that regard, the terms "instructions," "steps" and "computer program" may be used interchangeably herein. The instructions may be stored in object code form for direct processing by the processor, or in any other computer language including scripts or collections of independent source code modules that are interpreted on demand or compiled in advance.

In a preferred embodiment, of the computer implemented method of the invention, the expression levels of the biomarkers of the invention are received directly from equipment used for determining the expression levels. The expression levels of the biomarkers can be quantified as previously defined in the method of the invention.

In a further aspect, the invention refers to a system for the diagnosis of EoE, hereinafter "the system of the invention", comprising:

> i) a device that quantifies the expression levels of the biomarkers of the invention; and
> ii) at least one processor configured to carry out the computer implemented method of the invention; and
> iii) a display.

Preferably, the device that quantifies the expression levels of the biomarkers of the invention is a PCR equipment.

In a further aspect, the invention refers to a computer program adapted to carry out the steps of the computer implemented method of the invention, preferably using the system of the invention.

The implementation of the use and methods of the invention include the use of probes, primers and/or antibodies that can be part of a kit.

Thus, another aspect of the present invention refers to a kit, hereinafter "the kit of the invention", suitable for the diagnosis of EoE that comprises probes, primers and/or antibodies for identifying and quantifying the expression levels of CDH26, KCNJ2 and PLD1 biomarkers in a saliva sample isolated from a subject. The terms "CDH26, KCNJ2 and PLD1 biomarkers" have been defined in previous paragraphs.

In a preferred embodiment, the kit of the invention comprises the probes comprising, preferably consisting of, the following sequences SEQ ID NO: 4, SEQ ID NO: 7 and/or SEQ ID NO: 10. In a more preferred embodiment, alone or in combination with all or each one of the previous preferred embodiments, the kit of the invention comprises the primers comprising, preferably consisting of, the following sequences SEQ ID NO: 5, SEQ ID NO: 6, SEQ ID NO: 8, SEQ ID NO: 9, SEQ ID NO: 11 and/or SEQ ID NO: 12.

In the present invention, "probe" is understood as the nucleic acid molecule whose nucleotide sequence specifically hybridizes with the nucleotide sequence of the target biomarker. The term "specifically hybridizes", as used herein, refers to the conditions that allows the hybridization of two polynucleotides in highly or moderately demanding conditionals. High stringency conditions involve, in general: low ionic strength and high temperatura for washing, for example 0.015M sodium chloride/0.0015M sodium citrate/0.1% sodium dodecyl sulfate 50oC, (2) use during the hybridization of a denaturing agent, stories like formamide, e.g., 50% (v/v) formamide with 0.1% serum albumin bovine/0.1% Ficoll/0.1% polyvinylpyrrolidone/50 mM sodium phosphate buffer at pH 6.5 with 750 mM sodium chloride, 75 mM sodium citrate at 42°C, or (3) use of 50% formamide, 5xSSC (0.75M NaCl, 0.075M sodium citrate), 50mM sodium phosphate (pH 6.8), 0.1% sodium pyrophosphate, 5 times Denhardt's solution, sperm DNA from sonicated salmon (50 mg/mL), 0.1% SDS, and 10% dextran sulfate at 42oC, with washed at 42°C in 0.2xSSC (sodium chloride/sodium citrate) and 50% formamide, followed by a high stringency wash consisting of 0.1×SSC containing EDTA at 55°C. "Moderately stringent conditions" include the use of wash solution and the hybridization conditions less stringent than those described above.

In a preferred embodiment of the kit of the invention, the probes are dual -labelled probes. The term "dual-labelled probe" refers to a sequence of single-stranded oligonucleotides that have a marker, a quencher (marker that absorbs the signal from another marker) and a probe sequence arranged between the two markers, and in which marker and quencher are close enough as for the quencher to prevent the marker from emitting a detectable signal. The probe sequence usually includes a sequence that is sensitive to Taq polymerase. Typically, the probe comprises 20-30 nucleotides, preferably 20- 24 nucleotides. Marker molecules and quenching molecules are commercially available. Some examples are, but not limited to, the marker molecule 6-carboxifluorescein and the quenchers Iowa Black (IABkFQ) and ZEN of the company *Integrated DNA Technologies.*

In another preferred embodiment of the kit of the invention, the probes are triply probes marked. The term "triple-labeled probes" refers to a probe double marked that also contains a second internal quencher located between nucleotides 9 and 10 of the probe sequence. A non-limiting example of an internal quencher is the ZEN of the company *Integrated DNA Technologies.*

In addition to probes, primers and/or antibodies, the kit may comprise other components useful in the implementation of the present invention, such as, buffers, delivery vehicles, material carriers, positive control components and/or negative, etc. Optionally, such controls comprise the probes, primers and/or antibodies with modifications for use as controls, eg, probes, primers and/or antibodies that do not recognize the biomarkers of the invention. In addition to the components mentioned, the kits may also include instructions to practice the object of the invention. These instructions may be present in the kits mentioned in a variety of shapes, one or more of which may be present in the kit. Form in which these instructions may be present is as information printed on a suitable medium or substrate, e.g., a sheet or sheets of paper on which you print the information, on the kit packaging, on a package insert, etc. Another means would be computer-readable medium, for example, a CD, a USB, etc., on which the registered information. Another means that may be present is a site address that can be used over the Internet to access information on a remote site. Any convenient medium may be present in the kits.

In another aspect, the present invention refers to the use of the kit of the invention for the *in vitro* diagnosis of EoE. The terms "diagnosis" and "EoE" has been defined in previous paragraphs.

## DESCRIPTION OF THE DRAWINGS

**Fig. 1.** Differential expression of *CDH26, KCNJ2, PLD1, CRYAB, FAM43A, KCNJ2-AS1, LINC00707, SDR9C7* genes between active EoE patients (n=50) and controls (n=40) (validation cohort, cohort 3). *p<0.05, **p<0.01, ***p<0.001 by Student t-test.

**Fig. 2.** Differential expression of *CDH26, KCNJ2, PLD1, CRYAB, FAM43A, KCNJ2-AS1, LINC00707, SDR9C7* genes between active EoE patients (n=50) and EoE patients with clinical remission (n=24) (validation cohort, cohort 3).

**Fig. 3.** Individual Receiver Operating Characteristic (ROC) curves of *CDH26, KCNJ2* and *PLD1* (n=90).

**Fig. 4. A)** Combined P values (EoE score) of genes *CDH26, KCNJ2* and PLD1. **B)** ROC-plot of the combined prediction model (*CDH26 + KCNJ2* + PLD1) based on gene expression. *p<0.05, **p<0.01, ***p<0.001 by Student t-test.

**Fig. 5. A)** Combined P values (EoE score) of genes *CDH26, KCNJ2* and *PLD1* in addition to clinical data. **B)** ROC-plot of the combined prediction model based on gene expression including clinical data (*CDH26 + KCNJ2* + PLD1+ clinical data). *p<0.05, **p<0.01, ***p<0.001 by Student t-test.

### Examples

**Example 1.** Selection and validation of potential biomarkers for the diagnosis of EoE

#### Material and methods

#### Enrolment

The enrolment process was carried out by the "Servicio de Gastroenterologia del Hospital Universitario Donostia y del Hospital General de Tomelloso", wherein a screening of potential patients for their participation in the study was performed.

Adult volunteers who met the required characteristics were enrolled. necessary to carry out the study.

The inclusion/exclusion criteria for the enrolment of participants in the EoE cohort were the next:

Inclusion criteria:

- Patients who were diagnosed with Eosinophilic Esophagitis (EoE), who have presented symptoms of esophageal and who have at least 15 eosinophils per field in the histological biopsies of the esophagus.
- Having documented the exclusion of pathological infiltration by eosinophils in biopsy samples obtained from the gastric and duodenal mucosa.
- Exclusion of additional causes that might produce eosinophilic infiltration in the esophageal mucosa (e.g. Crohn's disease with esophageal involvement, hypereosinophilic syndrome, drug hypersensitivity, vasculitis, pemphigoid, connective tissue disorder, parasitosis or graft-versus-host disease)

Exclusion criteria:

- Evidence of different causes of EoE for esophageal eosinophilia
- Esophageal surgery at any time or esophageal dilation procedures in the last 8 weeks before the detection.
- Any relevant systemic disease such as ischemic heart disease, renal failure, chronic lung disease, chronic liver disease, neoplasms, etc, which, in the criteria of the physician, could jeopardized patient's wellbeing.
- Patients receiving systemic glucocorticosteroids, immunosuppressants, biological drugs, topical glucocorticosteroids, for other types of disease.

Those interested in participating in the study and who fulfilled the inclusion criteria, were informed orally and in writing of the relevant data of the study and decide whether or not to participate once they have clear knowledge of the study.

In addition, healthy voluntaries (testing cohort 1) or patients referred to upper endoscopy exams due to symptoms referred to the esophagus or upper gastrointestinal tract with no esophageal features of EoE (testing cohort 2 and validations cohort) were recruited. Esophageal biopsies were obtained from them by following the same protocol described for EoE and eosinophilic infiltration was excluded in all patients.

Permission to conduct the study was processed by the Research Ethics Committee of the Gipuzkoa Health Area (Protocol Code: BUJ-BIO-2020-01), and by the Committee of Clinical Research Ethics of the Hospital General La Mancha Centro (Code of protocol 137-C). All patients, control subjects, or their legal guardians, provided informed consent to participate in this study.

**Data Collection**

In all included patients, in addition to the usual clinical data of demographic information, data from the endoscopic report and pathological anatomy were also collected. The maximum number of eosinophils was counted in any of the points of the esophagus.

The results of endoscopy of the esophagus are classified according to the endoscopic reference score classification system (EREFS), adding the capacities of the 5 main fields (edema, exudates, furrows, strictures and rings). A total score and a global evaluation of the endoscopic activity of EoE were made and were classified as none, mild, moderate, or severe.

After collecting data from the patients, they were grouped into different cohorts according to table 1.

Table 1. Clinical characteristics of patients included in the study. Mean and standard deviation of number of cells/HPF and years are shown for peak eosinophils counts and age, respectively.

| Cohort | Dx | Eosinophil counts | Age | Sex | Related atopies | Kind of atopy |
|---|---|---|---|---|---|---|
| Testing cohort 1 (n=20) | EoE (n=10) | 50±22 | 41±15 | Male 80.0%<br><br>Female 20.0% | At least one atopy 40.0%<br><br>More than one atopy 30.0%<br><br>No atopies 60.0% | Asthma 10.0% Rhinitis 20.0% Conjunctivitis 10.0% Dermatitis 10.0% Food allergies 10.0% Non-food allergies 20.0% |
| | Controls (n=10) | N/A | 37±14 | Male 30.0% Female 70.0% | No atopies 100.0% | - |
| Testing cohort 2 (n=40) | Active EoE (n=19) | 61±37 | 33±13 | Male 78.9% | At least one atopy 78.9% | Asthma 26.3% |

(continued)

| Cohort | Dx | Eosinophil counts | Age | Sex | Related atopies | Kind of atopy |
|---|---|---|---|---|---|---|
| | | | | Female 21.1% | More than one atopy 52.6%<br><br>No atopies 21.1% | Rhinitis 57.9% Conjunctivitis 52.6% Dermatitis 5.3% Food allergies 42.8% |
| | Controls (n=13) | 0 | 34±16 | Male 38.5%<br><br>Female 61.5% | At least one atopy 7.7% More than one atopy 7.7% No atopies 92.3% | Asthma 15.4%<br><br>Rhinitis 7.7% |
| | EoE in remissio n (n=8) | 6±5 | 37±14 | Male 62.5%<br><br>Female 37.5% | At least one atopy 37.5% More than one atopy 12.5% No atopies 60.5% | Asthma 25.0% Rhinitis 25.0% Conjunctivitis 12.5% Food allergies 12.5% |
| Validatio n cohort (cohort 3) (n=114) | Active EoE (n=50) | 52±33 | 31±14 | Male 80.0%<br><br>Female 20.0% | At least one atopy 80.0%<br><br>More than one atopy 60.0%<br><br>No atopies 20.0% | Asthma 38.0% Rhinitis 52.0% Conjunctivitis 40.0% Dermatitis 20.0% Food allergies 30.0% Non-food allergies 10.0% |
| | Controls (n=40) | 0 | 47±13 | Male 47.5%<br><br>Female 52.5% | At least one atopy 22.5%<br><br>More than one atopy 7.5%<br><br>No atopies 77.5% | Asthma 7.5% Rhinitis 5.0% Conjunctivitis 2.5% Dermatitis 2.5% Non-food allergies 12.5% |

(continued)

| Cohort | Dx | Eosinophil counts | Age | Sex | Related atopies | Kind of atopy |
|---|---|---|---|---|---|---|
| | EoE in remissio n (n=28) | 6±6 | 34±15 | Male 70.8%<br><br><br>Female 29.2% | At least one atopy 87.5%<br><br>More than one atopy 70.8%<br><br>No atopies 12.5% | Asthma 41.7% Rhinitis 54.2% Conjunctivitis 58.3% Dermatitis 16.7% Food allergies 33.3% Non-food allergies 12.5% |

**Sample collection**

Saliva samples were collected from EoE cases and healthy controls. The used cases were patients diagnosed by histology with esophagitis eosinophilia without treatment or without response to treatment. The controls did not present any inflammatory disease. Saliva samples from each participant collected using the DNA/RNA Shield™ Collection Tube w/ Swab kit (Zymo) following the manufacturer's instructions.

**Selection of genes (biomarkers)**

Normalized gene expression data was retrieved from RNAseq study GSE113341 deposited in Gene Expression Omnibus (GEO) database. Normalized expression levels of 25474 genes were compared for differential expression using Mann-Whitney U-test and the significant genes, after False Discovery Rate test correction, were used for experimental biomarker validation. Only those genes included in the table 2 showed differential expression.

Table 2. Genes differently expressed

| Gene | Refseq | Fold change | p-value | FDR | Biological Process |
|---|---|---|---|---|---|
| CD200R1 | NM_138806.4, NM_138939.3, NM_138940.3, NM_170780.3 | 1,6867 | 1,30E-04 | 0,0480 | Cell adhesion |
| CD9 | NM_001769.4 | 0,6167 | 2,17E-05 | 0,0204 | Cell adhesion |
| CDH26 | NM_177980.4 | 3,2068 | 1,08E-05 | 0,0204 | Cell adhesion |
| CLDN22 | NM_001111319.3 | 2,7538 | 4,33E-05 | 0,0276 | Cell adhesion |
| DSG1 | NM_001942.4 | -2,6233 | 1,08E-05 | 0,0204 | Cell adhesion |
| MPP7 | NM_173496.5 | 0,5064 | 1,30E-04 | 0,0480 | Cell adhesion |
| ICAM4 | NM_001039132.3, NM_001544.5, NM_022377.3 | 1,6089 | 1,08E-05 | 0,0204 | Cell adhesion |
| CRYAB | NM_001885.3 | -1,0316 | 4,33E-05 | 0,0276 | Cell structure |
| DCN | NM_001920.5 | -0,7309 | 7,58E-05 | 0,0394 | Cell structure |

(continued)

| Gene | Refseq | Fold change | p-value | FDR | Biological Process |
|---|---|---|---|---|---|
| KRT32 | NM_002278.3 | -1,0753 | 2,17E-05 | 0,0204 | Cell structure |
| NAV3 | NM_014903.6 | -0,9481 | 1,30E-04 | 0,0480 | Cell structure |
| PDZD8 | NM_173791.5 | -0,3716 | 2,17E-05 | 0,0204 | Cell structure |
| SCIN | NM_001112706.3 | 1,1761 | 2,17E-05 | 0,0204 | Cell structure |
| HIPK3 | NM_004827.3 | -0,2058 | 7,58E-05 | 0,0394 | Gene expression regulation |
| RARB | NM_000965.5 | 1,1005 | 2,17E-05 | 0,0204 | Gene expression regulation |
| SA TB 1 | NM_001195470.3, NM_002971.6 | 0,3617 | 1,30E-04 | 0,0480 | Gene expression regulation |
| ZFC3H1 | NM_144982.5 | 0,3082 | 1,08E-05 | 0,0204 | Gene expression regulation |
| CCL24 | NM_002991.3 | 2,0312 | 7,58E-05 | 0,0394 | Immune response |
| CCL26 | NM_0060721.4 | 4,7290 | 1,08E-05 | 0,0204 | Immune response |
| CLNK | NM_052964.4 | 1,1749 | 1,08E-05 | 0,0204 | Immune response |
| ABHD4 | NM_022060.3 | 0,7329 | 4,33E-05 | 0,0276 | Metabolic process |
| CLN5 | NM_006493.4 | 0,5103 | 7,58E-05 | 0,0394 | Metabolic process |
| ENC1 | NM_001256574.2, NM_001256575.2, NM_001256576.2, NM_003633.4, NR_046318.2 | -0,5214 | 1,30E-04 | 0,0480 | Metabolic process |
| GCNT2 | NM_001491.3, NM_145655.4 | 0,8878 | 1,08E-05 | 0,0204 | Metabolic process |
| MINA | NM_001042533.3, NM_001261829.2, NM_032778.6, NM_153182.4 | -0,3088 | 1,30E-04 | 0,0480 | Metabolic process |
| OGFOD1 | NM_018233.4 | -0,2721 | 1,08E-05 | 0,0204 | Metabolic process |
| PLAA | NM_001031689.3 | -0,2100 | 1,30E-04 | 0,0480 | Metabolic process |
| PLD1 | NM_001130081.3, NM_002662.5 | -0,2612 | 1,30E-04 | 0,0480 | Metabolic process |
| RNF144A | NM_014746.6 | 0,4939 | 1,30E-04 | 0,0480 | Metabolic process |

(continued)

| Gene | Refseq | Fold change | p-value | FDR | Biological Process |
|---|---|---|---|---|---|
| SDR9C7 | NM_148897.3 | -0,9606 | 1,30E-04 | 0,0480 | Metabolic process |
| ABCG2 | NM_004827.3 | -0,8166 | 7,58E-05 | 0,0394 | Signalling |
| CACNB4 | NM_001005747.4 | 1,0464 | 1,30E-04 | 0,0480 | Signalling |
| CAPN14 | NM_001145122.2 | 1,5117 | 1,08E-05 | 0,0204 | Signalling |
| CAPN3 | NM_000070.3, NM_024344.2, NM_173087.2 | 0,5623 | 1,08E-05 | 0,0204 | Signalling |
| CARD9 | NM_052813.5, NM_052814.4 | 0,5692 | 1,30E-04 | 0,0480 | Signalling |
| CAST | NR_033798.1 | -0,6325 | 1,30E-04 | 0,0480 | Signalling |
| CDKN2AIP | NM_017632.4 | 0,5718 | 1,30E-04 | 0,0480 | Signalling |
| EDAR | NM_022336.4 | 1,2638 | 1,08E-05 | 0,0204 | Signalling |
| EPGN | NM_001013442.2 | -1,0461 | 1,08E-05 | 0,0204 | Signalling |
| FAM43A | NM_153690.5 | -0,7873 | 1,30E-04 | 0,0480 | Signalling |
| FAM83E | NM_017708.4 | 1,1390 | 7,58E-05 | 0,0394 | Signalling |
| FAM84B | NM_174911.5 | 0,5689 | 4,33E-05 | 0,0276 | Signalling |
| GCET2 | NM_001190259.2, NM_001190260.2, NM_152785.5 | 0,6040 | 1,30E-04 | 0,0480 | Signalling |
| GCNT2 | NM_145655.4 | 1,0718 | 2,17E-05 | 0,0204 | Signalling |
| HOMER1 | NM_004272.5 | -0,3549 | 2,17E-05 | 0,0204 | Signalling |
| KCNJ2 | NM_000891.3 | 1 ,7163 | 4,33E-05 | 0,0276 | Signalling |
| KIAA1609 | NM_001129993.3 | 0,3273 | 4,33E-05 | 0,0276 | Signalling |
| LRIG2 | NM_014813.3 | 0,1331 | 2,17E-05 | 0,0204 | Signalling |
| MAP3K13 | NM_004721.5 | 0,6545 | 2,17E-05 | 0,0204 | Signalling |
| P2RY1 | NM_002563.5 | 0,6521 | 1,08E-05 | 0,0204 | Signalling |
| PAMR1 | NM_001001991.3, NM_015430.4 | 1,3935 | 1,30E-04 | 0,0480 | Signalling |

(continued)

| Gene | Refseq | Fold change | p-value | FDR | Biological Process |
|---|---|---|---|---|---|
| PLK2 | NM_001252226.2, NM_006622.4 | -0,5631 | 4,33E-05 | 0,0276 | Signalling |
| SCUBE2 | NM_001170690.3, NM_020974.4 | 1,8356 | 4,33E-05 | 0,0276 | Signalling |
| SH2D4A | NM_001174160.2, NM_022071.4 | 0,3865 | 2,17E-05 | 0,0204 | Signalling |
| GRB14 | NM_0004490.3 | -0,6939 | 4,33E-05 | 0,0276 | Transport process |
| HEATR3 | NM_182922.4 | -0,3052 | 7,58E-05 | 0,0394 | Transport process |
| SLC2A12 | NM_145176.3 | 0,6372 | 2,17E-05 | 0,0204 | Transport process |
| SLC9A3 | NM_004174.4 | 3,5371 | 2,17E-05 | 0,0204 | Transport process |
| KCNJ2-AS1 | NR_036534.1 | 1,3979 | 4,33E-05 | 0,0276 | Uncharacterized ncRNA |
| LINC00346 | NR_ 027701.1 | -0,6052 | 4,33E-05 | 0,0276 | Uncharacterized ncRNA |
| LOC 100505782 | NR_040111.1 | -0,6140 | 4,33E-05 | 0,0276 | Uncharacterized ncRNA |
| LOC 100507127 | NR_038291.1 | 2,8311 | 1,30E-04 | 0,0480 | Uncharacterized ncRNA |
| LOC145837 | NR_026979.1 | 1,6894 | 7,58E-05 | 0,0394 | Uncharacterized ncRNA |
| LOC153684 | NR_015447.1 | 0,9129 | 2,17E-05 | 0,0204 | Uncharacterized ncRNA |
| MGC16275 | NR_026914.1 | 0,6606 | 4,33E-05 | 0,0276 | Uncharacterized ncRNA |
| NAGPA-AS1 | NR_038913.1 | 0,9690 | 1,30E-04 | 0,0480 | Uncharacterized ncRNA |
| PRICKLE2-AS1 | NR_045697.1 | Inf | 6,39E-05 | 0,0394 | Uncharacterized ncRNA |
| KIAA1841 | NM_001129993.3 | 0,3452 | 1,30E-04 | 0,0480 | Unkonwn |
| PRR15L | NM_024320.4 | 0,5388 | 2,17E-05 | 0,0204 | Unkonwn |

**RNA extraction and gene expression analysis**

Total RNA was extracted using trizol (Zymo Research, #R2053). 50-150ng of RNA was used for quantitative reverse transcription PCR (qRT-PCR) using PrimeTime™ One-Step RT-qPCR Master Mix (IDT, Inc., Coralville, Iowa, USA #10007067) and predesigned qPCR Assays for RNA quantitation (IDT, Inc., Coralville, Iowa, USA). Reactions were run in duplicate in a BioRad CFX384 real time PCR instrument. Expression levels were analysed using the 2-ΔΔCt method. The housekeeping genes analysed were *GAPDH, RPLP0* and *18S,* and the least variable was selected for relative expression normalization. All the candidate genes analysed and used assays and their sequences are available upon request.

**Predictive model generation**

Combinatorial analysis of the significant genes was performed using logistic regression formulas, Combined P values (EoE score) were obtained by an online logistic regression calculator (AAT Bioquest, Inc. (2022, April 13). *Quest Graph™ Logistic Regression (Logit) Calculator.* AAT Bioquest and were used for combined ROC curve analysis.
Clinical data, added to the model was first given a numeric value when needed. Age values were considered the exact age when the oral cavity samples were collected; sex punctuation was divided in 1 for male and 0 for female; and in the field of related atopies a value of 1 was given if patients had asthma, rhinitis, conjunctivitis, dermatitis and food and other kind allergies, and a 0 was given if not.

**Statistical Analysis**

Differential expression was considered significant when $p<0.05$ using a Student t-test. Outlier values were discarded using ROUT method with a Q of 1%.

**Results**

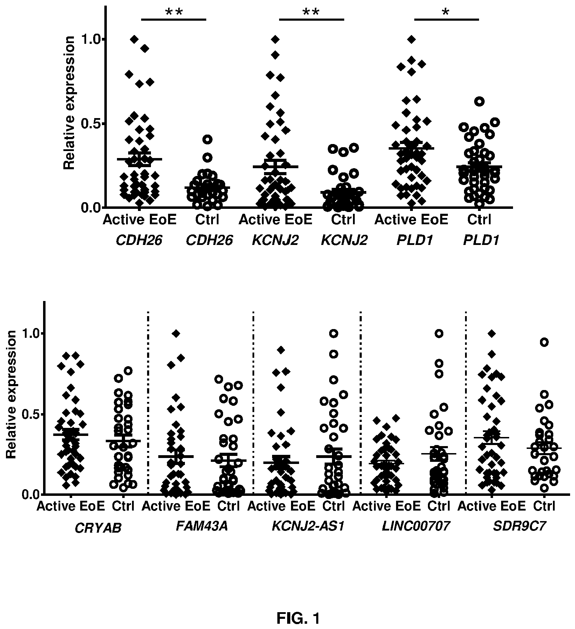

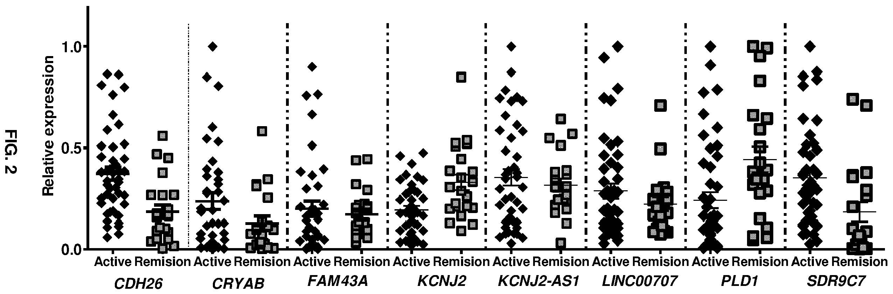

Expression of the candidate genes (table 2) and housekeeping genes was first quantified in oral cavity samples from 10 EoE patients and 10 controls (cohort 1). Genes for which amplification was observed in more than half of the samples, were selected for further analyses in a larger cohort of active EoE patients (n=19), controls (n=13) and EoE patients in clinical remission (n=8) (cohort 2). A total of 30 genes were analyzed: 27 genes were protein-coding genes, and 2 genes were long noncoding RNAs (lncRNAs). *GAPDH* was used as endogenous housekeeping gene. The eight most significant genes were selected for further potential biomarker validation.
For the validation phase, the expression of six coding genes (CDH26, CRYAB, FAM43A, KCNJ2, PLD1, SDR9C7) and two lncRNAs (LINC00707 and KCNJ2-AS1) was evaluated in oral samples from 50 active EoE patients, 40 controls and 24 EoE patients in clinical remission (Validation cohort, cohort 3) by qPCR using the primers and probes of table 3.
Those genes that showed differential expression between active EoE patients and controls of the cohort 3, specifically *CDH26, KCNJ2* and *PLD1 genes* (Fig. 1), were evaluated as possible non-invasive biomarkers for EoE diagnosis by calculating the receiver operating characteristic (ROC) curves. CRYAB, FAM43A, SDR9C7, LINC00707 and KCNJ2-AS1 did not show differential expression between active EoE patients and controls of the cohort 3. No differential expression was observed in any of these 8 genes when active EoE patients were compared with patients in clinical remission (Fig. 2).

Table 3 primers and probes for qPCR assay

| Gene | Type | Sequence |
|---|---|---|
| CDH26 | Probe | 56-FAM/TAC CCA GAT /ZEN/GCC ACA ATG CAC AGA /3IABkFQ (SEQ ID NO: 4) |
| | Primer 1 | GTC TCT GCC ATC CTT CCT TC (SEQ ID NO: 5) |
| | Primer 2 | GCA AGC CCT TTG AGC CAA (SEQ ID NO: 6) |
| KCNJ2 | Probe | 56-FAM/CCA CTT CCA /ZEN/CTC CAT GTC CCC A/3IABkFQ (SEQ ID NO: 7) |
| | Primer 1 | CAG CTG ACA TCC AGA GAA CA (SEQ ID NO: 8) |
| | Primer 2 | GCT CAC TCG CTT TTT ACA AAC C (SEQ ID NO: 9) |

(continued)

| Gene | Type | Sequence |
|---|---|---|
| PLD1 | Probe | 56-FAM/TGC TGC TGA /ZEN/TTG GTC TGC TGG TAT /3IABkFQ (SEQ ID NO: 10) |
|  | Primer 1 | GCC TGC TGT TCT CTA TCA CAT (SEQ ID NO: 11) |
|  | Primer 2 | GTC TGT CCA TGC TAA CGT ACA (SEQ ID NO: 12) |
| CRYAB | Probe | 56-FAM/AGC ACT TCT /ZEN/CCC CAG AGG AAC TCA /3IABkFQ (SEQ ID NO: 13) |
|  | Primer 1 | CAA TCA CAT CTC CCA ACA CCT (SEQ ID NO: 14) |
|  | Primer 2 | CTG GTT TGA CAC TGG ACT CTC (SEQ ID NO: 15) |
| FAM43A | Probe | 56-FAM/TTC CCC TCT /ZEN/CAA ATC TCC CCG C/3IABkFQ (SEQ ID NO: 16) |
|  | Primer 1 | AAC TTC AGC CAA CCT TCC C (SEQ ID NO: 17) |
|  | Primer 2 | CAC TTT TGG CTC TGT TGC G (SEQ ID NO: 18) |
| SDR9C7 | Probe | 56-FAM/CCC ACT TTG /ZEN/TCC CTC ACC CAC T/3IABkFQ (SEQ ID NO: 19) |
|  | Primer 1 | CAC CAG CAT TGT TCA CCA G (SEQ ID NO: 20) |
|  | Primer 2 | GGA TGT CAC CAA GAG CGA AA (SEQ ID NO: 21) |
| LINC00707 | Probe | 56-FAM/ATG ATC ACA /ZEN/AGG TGA GGT CCC ACA AC/3IABkFQ (SEQ ID NO: 22) |
|  | Primer 1 | CAG ATG TGC AGG AGG TGT TAG (SEQ ID NO: 23) |
|  | Primer 2 | GAC TTT ACT GGC TTT CTT GCT C (SEQ ID NO: 24) |
| KCNJ2-AS1 | Probe | 56-FAM/CAG ATC CGC /ZEN/ACT TAC TCC TGC CTT /3IABkFQ (SEQ ID NO: 25) |
|  | Primer 1 | AAG TAC CTT GCT CAG TGC TAA G (SEQ ID NO: 26) |
|  | Primer 2 | CTC CCA TCC TCA TAT CCA CAC (SEQ ID NO: 27) |

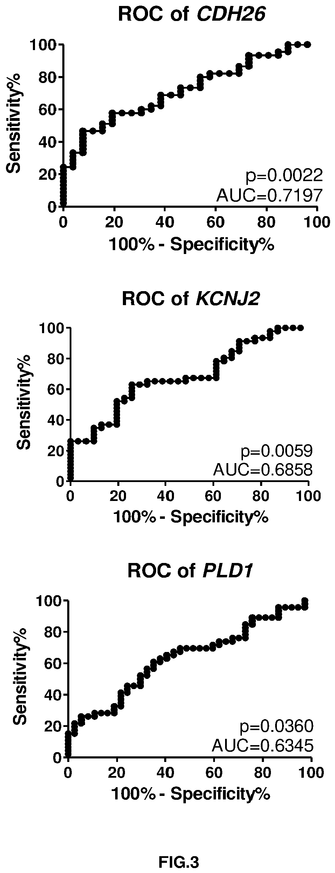

Individual evaluation of the diagnostic performance of the three significant genes using ROC analysis yielded an area under the curve (AUC) value of 0.72 for *CDH26* (p=0.0022), 0.69 for *KCNJ2* (p=0.0059), and 0.64 for *PLD1* (p=0.036) (Fig. 3).

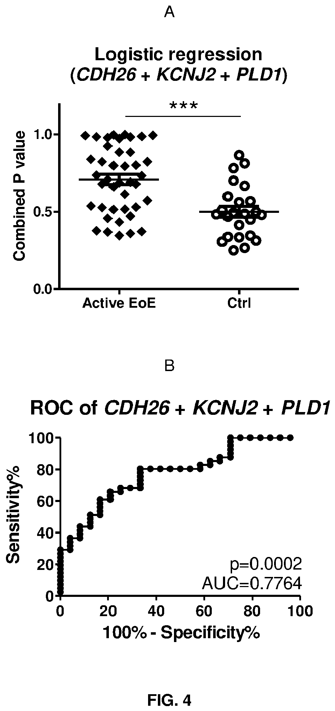

The diagnostic performance of the combination of those genes was also evaluated. Combined values were calculated by logistic regression formulas to obtain probability values (P values now on) and combined ROC curve analysis was used for to confirm discriminatory potential. These combined P values (EoE score) showed significant differences between active EoE patients and controls (Fig. 4A). The combination of *CDH26, KCNJ2* and *PLD1* yields a ROC-plot AUC of 0.78 (p=0.0002) (Fig. 4B), and the predictive model generated achieved 80.5% sensitivity and 66.7% specificity.

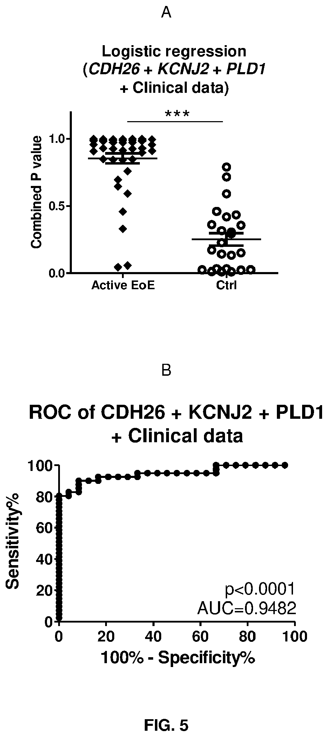

Additionally, adding clinical data such as age, sex, and the presence of related atopies significantly improved the predictive model. New combined P values of genes *CDH26, KCNJ2* and *PLD1* in addition to clinical data were calculated (Fig. 5A). Improved prediction model comprising oral biomarker expression and clinical data was also generated (Fig. 5B). This new combination yields a ROC-plot AUC of 0.95 (p<0.0001) (Figure 5B) with 90.2% sensitivity and 91.7% specificity.

Therefore, the inventors present a completely non-invasive approach for EoE diagnosis, based on objective and easy-to-collect clinical data and the expression of *CDH26, KCNJ2* and *PLD1* in the oral cavity. This combination efficiently works for initial diagnosis as it can distinguish active EoE patients from controls.

The prediction model developed by the inventors, showed high sensitivity and specificity scores, similar to the previously proposed EoE diagnostic panel (EDP) described in Wen T, et al. (2013). Gastroenterology, 145(6), 1289-1299, which is based on the gene expression profile of esophageal biopsies. However, the use of oral cavity samples instead of esophageal biopsies enables an easier and a much more comfortable procedure, in addition to being a faster and a more cost-effective way to diagnose EoE.

**Claims**

1. Use of the expression levels of CDH26, KCNJ2 and PLD1 genes as biomarkers for the *in vitro* diagnosis of eosinophilic esophagitis (EoE) in a saliva sample isolated from a subject.

2. Use according to claim 1, wherein the expression levels of CDH26, KCNJ2 and PLD1 biomarkers are combined with clinical data from the subject.

3. Use according to claims 1 or 2, wherein the expression levels of CDH26, KCNJ2 and PLD1 biomarkers are quantified by the expression levels of the messenger RNA (mRNA) of said biomarkers, or a fragment of said mRNA, of the complementary DNA (cDNA) of said biomarkers, or a fragment of said cDNA, or of the protein encoded by said biomarkers, or a fragment of said protein.

4. An *in vitro* method for the diagnosis of EoE in a subject, wherein said method comprises the following steps:

   a) quantifying the expression levels of CDH26, KCNJ2 and PLD1 biomarkers in a saliva sample isolated from the subject; and
   b) comparing the expression levels of each biomarker of step a) with reference values for said biomarkers;

   wherein an increase in the expression levels of CDH26, KCNJ2 and PLD1 biomarkers on the sample from the subject compared to reference values is indicative that the subject is suffering from, or is predisposed to suffering from, EoE.

5. A computer implemented method for the diagnosis of EoE, the computer performing the steps comprising:

   a) receiving inputted subject data comprising values for the expression levels of CDH26, KCNJ2 and PLD1 biomarkers in a saliva sample isolated from the subject; and
   b) calculating an EoE score, wherein EoE score is a result of applying a probability function to a linear combination of the expression levels;
   c) determining that the subject suffers from, or is predisposed to suffering from, EoE, if the EoE score is greater than or equal to a reference score.

6. A computer implemented method according to claim 5, the step a) further comprises receiving clinical data from the subject, wherein clinical data comprises at least the age, sex and/or the presence or absence of atopies, being the atopies selected from the list consisting of asthma, rhinitis, dermatitis, food allergy and non-food allergy; and wherein the EoE score calculated in step b) is the result of applying a probability function to a linear combination of the expression levels of CDH26, KCNJ2 and PLD1 biomarkers together with the inputted clinical data from the subject.

7. Method according to claim 6, wherein the probability function used is:

$$EoE\ score\ =\ 1/(1 + e^{\wedge}(-z))$$

   wherein z is the linear combination of the expression levels of CDH26, KCNJ2 and PLD1 biomarkers together with the inputted clinical data from the subject.

8. Method according to claim 7, wherein the z is calculated according to the formula:

$$Z = -2{,}2303 + (2{,}0286 * \text{CDH26}) + (5{,}809 * \text{KCNJ2}) + (3{,}210 * \text{PLD1}) - (0{,}049$$

$$* \text{Age}) + (2{,}1235 * \text{Sex}) + (2{,}7705 * \text{Presence of atopies})$$

9. Method according to claim 7, wherein the value of the reference score is 0.5898 or 0.815.

10. Method according to any one of claims 4 to 9, wherein the expression levels of CDH26, KCNJ2 and PLD1 biomarkers are quantified by (i) the expression levels of the messenger RNA (mRNA) of said biomarkers, or a fragment of said mRNA, or (ii) the complementary DNA (cDNA) of said biomarkers, or a fragment of said cDNA, or (iii) the protein encoded by said biomarkers, or a fragment of said protein.

11. Method according to claim 10, wherein the quantification of mRNA or cDNA levels is carried out by polymerase chain reaction; or wherein the quantification of the protein levels is carried out by Western blotting or enzyme-linked immunosorbent assay.

12. A kit for the *in vitro* diagnosis of EoE that comprises probes, primers and/or antibodies for identifying and quantifying the expression levels of CDH26, KCNJ2 and PLD1 biomarkers in a saliva sample isolated from a subject.

13. Use of the kit according to claim 12 for the *in vitro* diagnosis of EoE.

14. A system for the diagnosis of EoE comprising:

   i. a device that quantifies the expression levels of CDH26, KCNJ2 and PLD1 biomarkers; and
   ii. at least one processor configured to carry out the computer implemented method of the invention; and
   iii. a display

15. A computer program adapted to carry out the steps of computer implemented method according to any one of claims 5 to 9.

FIG. 1

FIG. 2

## ROC of *CDH26*

p=0.0022
AUC=0.7197

## ROC of *KCNJ2*

p=0.0059
AUC=0.6858

## ROC of *PLD1*

p=0.0360
AUC=0.6345

FIG.3

A

**Logistic regression
(*CDH26* + *KCNJ2* + *PLD1*)**

B

**ROC of *CDH26* + *KCNJ2* + *PLD1***

p=0.0002
AUC=0.7764

**FIG. 4**

A

**Logistic regression**
**(*CDH26* + *KCNJ2* + *PLD1***
**+ Clinical data)**

B

**ROC of CDH26 + KCNJ2 + PLD1**
**+ Clinical data**

p<0.0001
AUC=0.9482

**FIG. 5**

Europäisches
Patentamt

European
Patent Office

Office européen
des brevets

## EUROPEAN SEARCH REPORT

Application Number

EP 23 38 2070

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/069780 A1 (SERVICIO DE SALUD DE CASTILLA LA MANCHA [ES] ET AL.) 7 April 2022 (2022-04-07) * claims 1-4; figures 1-5; example 1 * | 1-15 | INV. C12Q1/6883 G01N33/53 |
| X | US 10 155 985 B2 (CHILDRENS HOSPITAL MED CT [US]) 18 December 2018 (2018-12-18) | 12,13 | |
| A | * table 1; sequences 6, 40, 1015 * * column 7, last paragraph – column 8 * * column 75, paragraph 2-4 * | 1-11,14, 15 | |
| A | STRAUMANN ALEX ET AL: "Lifting the Veil: The Quest for Noninvasive Biomarkers for the Accurate Diagnosis of Eosinophilic Esophagitis", DIGESTIVE DISEASES AND SCIENCES, SPRINGER NEW YORK LLC, US, vol. 66, no. 5, 10 July 2020 (2020-07-10), pages 1388-1389, XP037427324, ISSN: 0163-2116, DOI: 10.1007/S10620-020-06451-8 [retrieved on 2020-07-10] * the whole document * | 1-15 | |

TECHNICAL FIELDS
SEARCHED      (IPC)

C12Q
G01N

The present search report has been drawn up for all claims

| Place of search | Date of completion of the search | Examiner |
|---|---|---|
| Munich | 23 June 2023 | Eveleigh, Anna |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................................

& : member of the same patent family, corresponding
document

EPO FORM 1503 03.82 (P04C01)

# EP 4 407 045 A1

## ANNEX TO THE EUROPEAN SEARCH REPORT
## ON EUROPEAN PATENT APPLICATION NO.

EP 23 38 2070

23-06-2023

| Patent document cited in search report | | Publication date | Patent family member(s) | | Publication date |
|---|---|---|---|---|---|
| WO 2022069780 | A1 | 07-04-2022 | ES 2902730 | A1 | 29-03-2022 |
| | | | WO 2022069780 | A1 | 07-04-2022 |
| US 10155985 | B2 | 18-12-2018 | US 2009233275 | A1 | 17-09-2009 |
| | | | US 2009269774 | A1 | 29-10-2009 |
| | | | US 2011195500 | A1 | 11-08-2011 |
| | | | US 2017067111 | A1 | 09-03-2017 |
| | | | WO 2006083390 | A2 | 10-08-2006 |

EPO FORM P0459

For more details about this annex : see Official Journal of the European Patent Office, No. 12/82

# EP 4 407 045 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2017006711 A
- US 20160304960 A
- WO 2015142739 A

### Non-patent literature cited in the description

- **DELLON, E. S. ; HIRANO, I.** *Gastroenterology,* 2018, vol. 154 (2), 319-332
- **DELLON et al.** *Gastroenterology.,* 2018, vol. 155 (4), 1022-1033
- **ALTSCHUL S.F. et al.** Basic local alignment search tool. *J Mol Biol.,* 05 October 1990, vol. 215 (3), 403-10
- *Allergy.,* 2001, vol. 56, 813-24
- **WEN T et al.** *Gastroenterology,* 2013, vol. 145 (6), 1289-1299